(19) Europäisches Patentamt
European Patent Office
Office européen des brevets

(11) Veröffentlichungsnummer: **0 433 804 A1**

# (12) EUROPÄISCHE PATENTANMELDUNG

(21) Anmeldenummer: **90123603.4**

(22) Anmeldetag: **08.12.90**

(51) Int. Cl.⁵: **C07D 249/12**, C07D 413/06, C07D 401/06, A01N 43/653, A01N 43/84

(30) Priorität: **21.12.89 DE 3942222**

(43) Veröffentlichungstag der Anmeldung: **26.06.91 Patentblatt 91/26**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Jelich, Klaus, Dr.**
**Paul-Ehrlich-Strasse 2**
**W-5600 Wuppertal 1(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim**
**Gruenstrasse 9a**
**W-5090 Leverkusen(DE)**
Erfinder: **Schmidt, Robert R., Dr.**
**Im Waldwinkel 110**
**W-5060 Bergisch Gladbach 2(DE)**

(54) **Substituierte Carbamoyltriazole.**

(57) Die Erfindung betrifft neue substituierte Carbamoyltriazole der Formel (I)

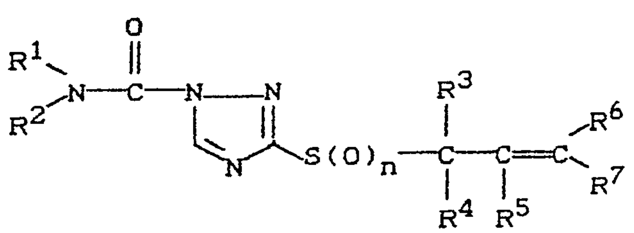

(I)

in welcher

n für die Zahlen 0, 1 oder 2 steht,

$R^1$ und $R^2$ gleich oder verschieden sind und unabängig voneinander jeweils für geradkettiges oder verzweigtes Alkyl, Alkenyl oder Alkinyl mit jeweils bis zu 6 Kohlenstoffatomen oder zusammen für jeweils geradkettiges oder verzweigtes Alkandiyl oder Oxaalkandiyl mit jeweils bis zu 6 Kohlenstoffatomen stehen,

$R^3$ und $R^4$ gleich oder verschieden sind und unabhängig voneinander jeweils für Wasserstoff oder für geradkettiges oder verzweigtes Alkyl mit bis zu 6 Kohlenstoffatomen stehen, und

$R^5$, $R^6$ und $R^7$ gleich oder verschieden sind und jeweils für Wasserstoff, Halogen, Cyano oder für jeweils geradkettiges oder verzweigtes Alkyl oder Halogenalkyl mit jeweils bis zu 6 Kohlenstoffatomen und gegebenenfalls bis zu 13 Halogenatomen stehen,

mit der Maßgabe, daß wenigstens einer der Reste $R^5$, $R^6$, $R^7$ für Halogen steht,
Verfahren zu ihrer Herstellung und ihre Verwendung als Herbizide.

EP 0 433 804 A1

## SUBSTITUIERTE CARBAMOYLTRIAZOLE

Die Erfindung betrifft neue substituierte Carbamoyltriazole, Verfahren zu ihrer Herstellung und ihre Verwendung als Herbizide.

Es ist bekannt, daß bestimmte substituierte Carbamoyltriazole herbizide Eigenschaften aufweisen (vgl. DE-OS 2 132 618). Die Wirkung dieser bekannten Verbindungen ist jedoch nicht in allen Belangen zufriedenstellend.

Es wurden nun die neuen substituierten Carbamoyltriazole dar allgemeinen Formel (I)

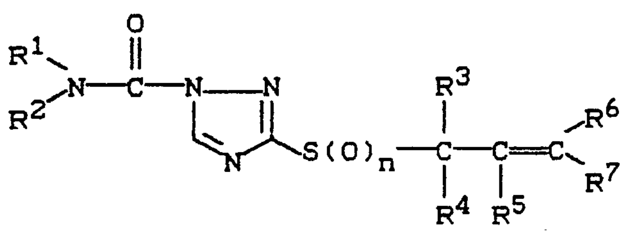

(I)

in welcher

n für die Zahlen 0, 1 oder 2 steht,

$R^1$ und $R^2$ gleich oder verschieden sind und unabängig voneinander jeweils für geradkettiges oder verzweigtes Alkyl, Alkenyl oder Alkinyl mit jeweils bis zu 6 Kohlenstoffatomen oder zusammen für jeweils geradkettiges oder verzweigtes Alkandiyl oder Oxaalkandiyl mit jeweils bis zu 6 Kohlenstoffatomen stehen,

$R^3$ und $R^4$ gleich oder verschieden sind und unabhängig voneinander jeweils für Wasserstoff oder für geradkettiges oder verzweigtes Alkyl mit bis zu 6 Kohlenstoffatomen stehen, und

$R^5$, $R^6$ und $R^7$ gleich oder verschieden sind und jeweils für Wasserstoff, Halogen, Cyano oder für jeweils geradkettiges oder verzweigtes Alkyl oder Halogenalkyl mit jeweils bis zu 6 Kohlenstoffatomen und gegebenenfalls bis zu 13 Halogenatomen stehen,

mit der Maßgabe, daß wenigstens einer der Reste $R^5$, $R^6$ und $R^7$ für Halogen steht, gefunden.

Weiter wurde gefunden, daß man die neuen Verbindungen der Formel (I) erhält, wenn man

(a) substituierte Triazole der allgemeinen Formel (II)

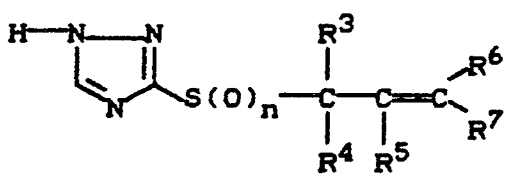

(II)

in welcher

n, $R^3$, R4, R5, R6 und $R^7$ die oben angegebenen Bedeutungen haben,

oder Tautomere zu den Verbindungen der Formel (II)

mit Carbamidsäurechloriden der allgemeinen Formel (III)

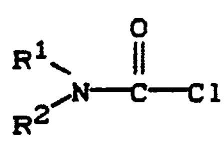

(III)

in welcher

$R^1$ und $R^2$ die oben angegebenen Bedeutungen haben,

gegebenenfalls in Gegenwart eines Säureakzeptors und gegebenenfalls in Gegenwart eines Verdünnungsmittels umsetzt, oder wenn man (b) substituierte Carbamoyltriazole der allgemeinen Formel (Ia)

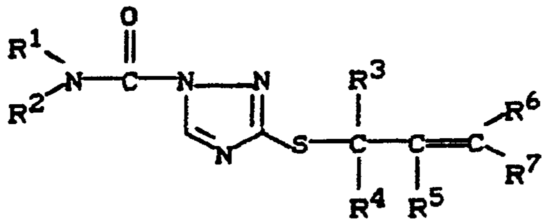

(Ia)

in welcher

$R^1$, $R^2$, $R^3$, $R^4$, $R^5$, $R^6$ und $R^7$ die oben angegebenen Bedeutungen haben, mit Oxidationsmitteln, gegebenenfalls in Gegenwart von Verdünnungsmitteln umsetzt.

Schließlich wurde gefunden, daß die neuen substituierten Carbamoyltriazole der allgemeinen Formel (I) interessante herbizide Eigenschaften aufweisen.

Die erfindungsgemäßen substituierten carbamoyltriazole sind durch die Formel (I) allgemein definierte.Bevorzugt sind die Verbindungen der Formel (I), bei welchen

n für die Zahlen 0, 1 oder 2 steht,

$R^1$ und $R^2$ gleich oder verschieden sind und unabhängig voneinander jeweils für geradkettiges oder verzweigtes Alkyl, Alkenyl oder Alkinyl mit jeweils

bis zu 4 Kohlenstoffatomen oder zusammen für Tetramethylen (Butan-1,4-diyl), Pentamethylen (Pentan-1,5-diyl) oder 3-Oxa-pentan-1,5-diyl ($-CH_2CH_2-O-CH_2CH_2-$) stehen,

$R^3$ und $R^4$ gleich oder verschieden sind und unabhängig voneinander jeweils für Wasserstoff oder für geradkettiges oder verzweigtes Alkyl mit bis zu 3 Kohlenstoffatomen stehen, und

$R^5$, $R^6$ und $R^7$ gleich oder verschieden sind und jeweils für Wasserstoff, Fluor, Chlor, Brom, Jod, Cyano oder für jeweils geradkettiges oder verzweigtes Alkyl oder für durch Fluor und/oder Chlor substituiertes Alkyl mit jeweils bis zu 4 Kohlenstoffatomen und gegebenenfalls bis zu 9 Fluor- und/oder Chloratomen stehen,

mit der Maßgabe, daß wenigstens einer der Reste $R^5$, $R^6$, $R^7$ für Fluor, Chlor, Brom oder Jod steht.

Besonders bevorzugt sind die Verbindungen der Formel (I), bei welchen

n für die Zahlen 0, 1 oder 2 steht,

$R^1$ und $R^2$ gleich oder verschieden sind und unabhängig voneinander jeweils für Methyl, Ethyl, Propyl, Isopropyl, Allyl oder Propargyl stehen,

$R^3$ für Wasserstoff oder Methyl steht,

$R^4$ für Wasserstoff, Methyl, Ethyl, Propyl oder Isopropyl steht,

$R^5$ für Wasserstoff, Chlor, Methyl oder Ethyl steht, und

$R^6$ und $R^7$ gleich oder verschieden sind und jeweils für Wasserstoff, Chlor, Methyl, Ethyl oder Propyl stehen,

mit der Maßgabe, daß wenigstens einer der Reste $R^5$, $R^6$, $R^7$ für Chlor steht.

Verwendet man beispielsweise 3-(3-Chlor-2-buten-1-yl-thio)-1H-1,2,4-triazol und N,N-Dimethyl-carbamidsäurechlorid als Ausgangsstoffe, so läßt sich der Reaktionsablauf beim erfindungsgemäßen Verfahren (a) durch das folgende Formelschema darstellen:

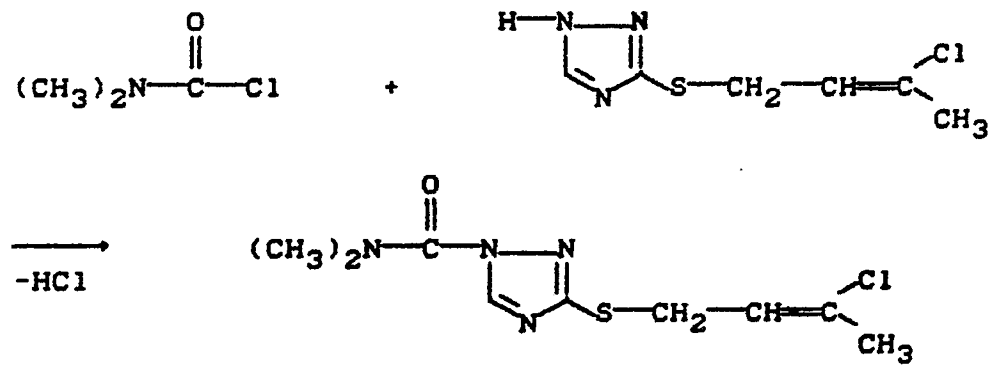


Verwendet man beispielsweise 1-(Dipropylaminocarbonyl)-3-(2,3-dichlor-2-penten-1-yl-thio)-1H-1,2,4-triazol und Hydrogenperoxid als Ausgangsstoffe, so läßt sich der Reaktionsablauf beim erfindungsgemäßen Verfahren (b) durch das folgende Formelschema darstellen:

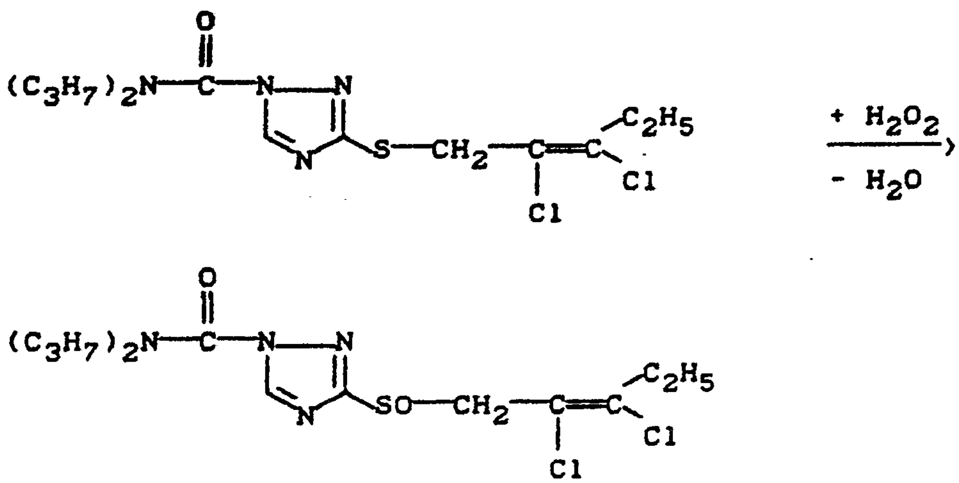


Die beim erfindungsgemäßen Verfahren (a) zur Herstellung von Verbindungen der Formel (I) als Ausgangsstoffe zu verwendenden substituierten Triazole sind durch die Formel (II) allgemein definiert.

In Formel (II) haben n, $R^3$, $R^4$, $R^5$, $R^6$ und $R^7$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, $R^3$, $R^4$, $R^5$, $R^6$ und $R^7$ angegeben wurden.

Beispiele für die Ausgangsstoffe der Formel (II) sind in der nachstehenden Tabelle 1 aufgeführt.

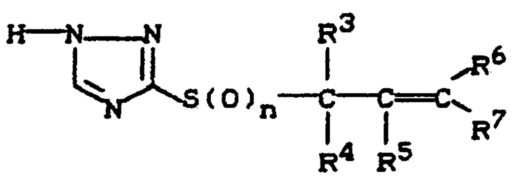

(II)

**Tabelle 1:** Beispiele für die Ausgangsstoffe der Formel (II)

| n | $R^3$ | $R^4$ | $R^5$ | $R^6$ | $R^7$ |
|---|---|---|---|---|---|
| 0 | H | H | Cl | H | H |
| 1 | H | H | Cl | H | H |
| 2 | H | H | Cl | H | H |
| 0 | H | H | H | Cl | H |
| 1 | H | H | H | Cl | H |
| 2 | H | H | H | Cl | H |
| 0 | H | H | H | Cl | Cl |
| 1 | H | H | H | Cl | Cl |
| 2 | H | H | H | Cl | Cl |
| 0 | H | H | H | Cl | $CH_3$ |
| 1 | H | H | H | Cl | $CH_3$ |
| 2 | H | H | H | Cl | $CH_3$ |
| 0 | H | H | $CH_3$ | H | Cl |
| 1 | H | H | $CH_3$ | H | Cl |
| 2 | H | H | $CH_3$ | H | Cl |
| 0 | H | $CH_3$ | H | H | Cl |

**Tabelle 1** - Fortsetzung

| n | $R^3$ | $R^4$ | $R^5$ | $R^6$ | $R^7$ |
|---|---|---|---|---|---|
| 1 | H | $CH_3$ | H | H | Cl |
| 2 | H | $CH_3$ | H | H | Cl |
| 0 | $CH_3$ | $CH_3$ | H | H | Cl |
| 1 | $CH_3$ | $CH_3$ | H | H | Cl |
| 2 | $CH_3$ | $CH_3$ | H | H | Cl |

Die Ausgangsstoffe der Formel (II) sind - mit der Maßgabe, daß wenigstens einer der Reste $R^5$, $R^6$, $R^7$ für Halogen steht - noch nicht aus der Literatur bekannt und Gegenstand der vorliegenden Erfindung.

Man erhält die neuen substituierten Triazole der Formel (II), wenn man das Mercaptotriazol der beiden tautomeren Formeln (IVa) und (IVb)

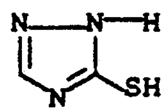

(IVa)

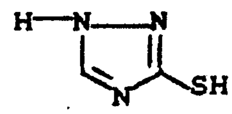

(IVb)

mit Alkenylhalogeniden der allgemeinen Formel (V)

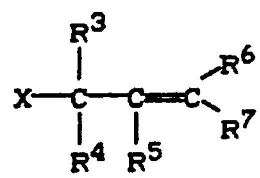

(V)

in welcher

$R^3$, $R^4$, $R^5$, $R^6$ und $R^7$ die oben angegebenen Bedeutungen haben und

X für Halogen steht,

gegebenenfalls in Gegenwart eines Säureakzeptors, wie z.B. Natriummethanolat, Natriumethanolat, Kaliumcarbonat oder Pyridin, und gegebenenfalls in Gegenwart eines Verdünnungsmittels, wie z.B. Methanol, Ethanol, Acetonitril oder Dimethylformamid, bei Temperaturen zwischen 0 °C und 100 °C umsetzt und nach üblichen Methoden aufarbeitet (vgl. die Herstellungsbeispiele) sowie gegebenenfalls die so erhaltenen Verbindungen der Formel (II), bei denen n für Null steht, wie für das erfindungsgemäße Verfahren (b) beschrieben oxidiert.

Die benötigten Vorprodukte der Formeln (IVa)/(IVb) und (V) sind bekannte organische Chemikalien.

Die beim erfindungsgemäßen Verfahren (a) weiter als Ausgangsstoffe zu verwendenden Carbamidsäurechloride sind durch die Formel (III) allgemein definiert.

In Formel (III) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Als Beispiele für die Ausgangsstoffe der Formel (III) seien genannt: N,N-Dimethyl-, N,N-Diethyl- und N,N-Dipropyl-carbamidsäurechlorid. Die Ausgangsstoffe der Formel (III) sind bekannte organische Chemikalien.

Die beim erfindungsgemäßen Verfahren (b) benötigten Ausgangsstoffe der Formel (Ia) sind erfindungsgemäße, neue Verbindungen; sie können nach dem erfindungsgemäßen Verfahren (a) hergestellt werden.

Das erfindungsgemäße Verfahren (a) zur Herstellung der neuen Verbindungen der Formel (I) wird gegebenenfalls unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden.

Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,-2]-octan (DABCO).

Soweit flüssige Säureakzeptoren, wie z.B. Pyridin, verwendet werden, können sie im Überschuß eingesetzt auch als Verdünnungsmittel dienen.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden (vgl. die Herstellungsbeispiele).

Das erfindungsgemäße Verfahren (b) wird unter Einsatz eines Oxidationsmittels durchgeführt. Es können die üblichen zur Oxidation von Sulfiden zu Sulfoxiden oder Sulfonen verwendbaren Oxidationsmittel verwendet werden. Hierzu gehören beispielsweise Hydrogenperoxid, Perameisensäure, Peressigsäure, Perbenzoesäure und 3-Chlor-perbenzoesäure.

Das erfindungsgemäße Verfahren (b) wird vorzugsweise unter Verwendung von Verdünnungsmitteln durchgeführt. Hierzu gehören organische Lösungsmittel wie Methylenchlorid, Chloroform, Ameisensäure, Essigsäure und Propionsäure, ferner auch Wasser.

Die Reaktionstemperaturen können bei dem erfindungsgemäßen Verfahren (b) in einem größeren Bereich variiert werden. Im allgemeinen arbeitet man bei Temperaturen zwischen -30 °C und +100 °C, vorzugsweise bei Temperaturen zwischen -10 °C und +80 °C.

Verfahren (b) wird im allgemeinen bei Normaldruck durchgeführt.

Zur Durchführung des erfindungsgemäßen Verfahrens (b) setzt man auf 1 Mol einer Verbindung der Formel (Ia) im allgemeinen 0,9 bis 1,5 Moläquivalente, vorzugsweise 1 bis 1,2 Moläquivalente eines Oxidationsmittels (zur Herstellung von Sulfoxiden) bzw. 1,9 bis 2,8 Moläquivalente, vorzugsweise 2 bis 2,5 Moläquivalente eines Oxidationsmittels (zur Herstellung von Sulfonen) ein.

Die Reaktionskomponenten werden im allgemeinen bei Raumtemperatur oder unter Kühlen zusammengegeben und das Reaktionsgemisch wird bei der jeweils erforderlichen Temperatur bis zum Ende der Umsetzung gerührt. Die Aufarbeitung erfolgt bei dem erfindungsgemäßen Verfahren (b) jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen</u>: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen</u>: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen</u>: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen</u>: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur

selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen, vor allem im Vorauflauf-Verfahren.

In gewissem Umfang zeigen die Verbindungen der Formel (I) auch fungizide Wirkung, insbesondere gegen Pyricularia oryzae am Reis und gegen Oomyceten.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z`B` Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden,

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z,B, 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methyl-thio-1,2,4-triazin-5(4H)-on (METRIBUZIN) zur Unkrautbekämpfung in Sojabohnen, in Frage; ferner auch Chloressigsäure-N-(methoxymethyl)-2,6-diethylanilid (ALACHLOR); 2-Chlor-4-ethylamino-6-isopropylamino-1,3,5-triazin (ATRAZIN); 3,5-Dibrom-4-hydroxy-benzonitril (BROMOXYNIL); Ethyl-2-{[(4-chlor-6-methoxy-2-pyrimidinyl)-aminocarbonyl]-aminosulfonyl}-benzoat (CHLORIMURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridin-carbonsäure (CLOPYRALID); 2-Chlor-4-ethylamino-6-(3-cyanopropylamino)-1,3,5-triazin (CYANAZIN); N,N-Di-n-propyl-thiocarbamidsäure-S-ethylester (EPTAME); 4-Amino-6-t-butyl-3-ethylthio-1,2,4-triazin-5(4H)-on (ETHIOZIN); 1-Methyl-3-phenyl-5-(3-trifluormethylphenyl)-4-pyridon (FLURIDONE); 3,5-Diiod-4-hydroxybenzonitril (IOXYNIL); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); (2-Chlor-4-trifluormethylphenyl)-(3-ethoxy-4-nitro-phe-nyl)-ether (OXYFLUORFEN); 2-Chlor-N-isopropylacetanilid (PROPACHLOR); 0-(6-Chlor-3-phenyl-pyridazin-4-yl)-S-octyl-thiocarbonat (PYRIDATE); 2-Chlor-4,6-bis-(ethylamino)-1,3,5,triazin (SIMAZIN); 2,4-Bis-[N-ethylaminol-6-methylthio-1,3,5-triazin (SIMETRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen,

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem großeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

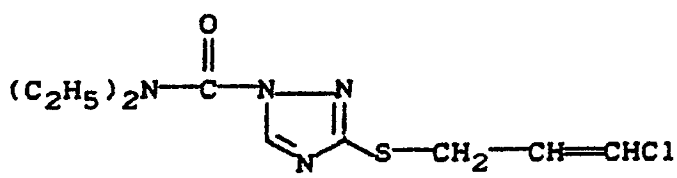

(Verfahren (a))

8,13 g (60 mMol) N,N-Diethylcarbamidsäurechlorid werden unter Rühren zu einer Mischung aus 8,0 g (45,6 mMol) 3-(3-Chlor-2-propen-1-yl-thio)-1H-1,2,4-triazol und 50 ml Pyridin tropfenweise gegeben und das Gemisch wird 20 Stunden bei 20 °C gerührt. Anschließend wird das Gemisch auf Eiswasser gegossen und mit Essigsäureethylester extrahiert. Die organische Phase wird mit 2N-Salzsäure gewaschen, mit Magnesiumsulfat getrocknet und filtriert. Vom Filtrat wird das Lösungsmittel im Wasserstrahlvakuum sorgfältig abdestilliert.

Man erhält 9,5 g (76% der Theorie) 1-(Diethylaminocarbonyl)-3-(3-chlor-2-propen-1-yl-thio)-1H-1,2,4-triazol als öligen Rückstand.

$^1$H-NMR ($CDCl_3$, δ, ppm): 3,65; 3,95.

Beispiel 2

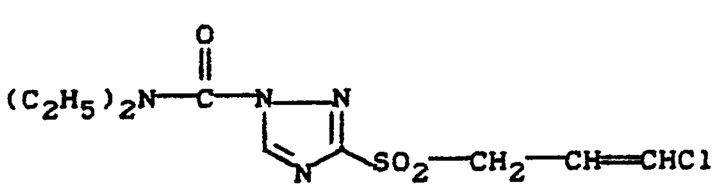

(Verfahren (b))

9,7 g (45 mMol) 3-Chlor-perbenzoesäure werden zu einer mit Eis gekühlten Mischung aus 5,0 g (20,4 mMol) 1-(Diethylaminocarbonyl)-3-(3-chlor-2-propen-1-yl-thio)-1H-1,2,4-triazol und 100 ml Chloroform unter Rühren portionsweise gegeben. Das Gemisch wird 2 Stunden weiter unter Eiskühlung und weitere 10 Stunden bei 20 °C gerührt. Anschließend wird filtriert, das Filtrat mit gesättigter Kaliumcarbonat-Lösung gewaschen, mit Magnesiumsulfat getrocknet und filtriert. Vom Filtrat wird das Lösungsmittel im Wasserstrahlvakuum sorgfältig abdestilliert.

Man erhält 6,0 g (96% der Theorie) 1-(Diethylaminocarbonyl)-3-(3-chlor-2-propen-1-yl-sulfonyl)-1H-1,2,4-triazol als öligen Rückstand.

$^1$H-NMR ($CDCl_3$, δ, ppm): 4,05; 4,30.

Analog zu den Beispielen 1 und 2 sowie entsprechend den allgemeinen Angaben zur Durchführung der erfindungsgemäßen Verfahren können beispielsweise auch die in der nachstehenden Tabelle 2 aufgeführten Verbindungen der Formel (I) hergestellt werden.

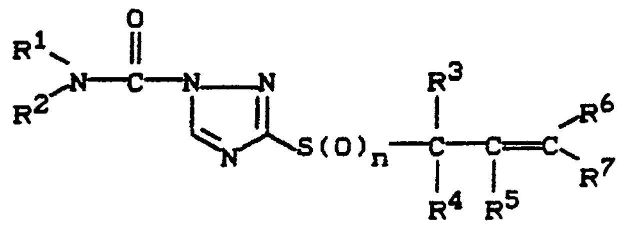

(I)

**Tabelle 2: Beispiele für die Verbindungen der Formel (I)**

| Bsp.-Nr. | n | $R^1$ | $R^2$ | $R^3$ | $R^4$ | $R^5$ | $R^6$ | $R^7$ | Physikalische Daten |
|---|---|---|---|---|---|---|---|---|---|
| 3 | 0 | $CH_3$ | $CH_3$ | H | H | H | Cl | H | $^1$H-NMR*): 3,65; 3,95 |
| 4 | 2 | $CH_3$ | $CH_3$ | H | H | H | Cl | H | $^1$H-NMR*): 4,15; 4,35 |
| 5 | 0 | $C_2H_5$ | $C_2H_5$ | H | H | H | Cl | Cl | $^1$H-NMR*): 3,85 |
| 6 | 0 | $CH_3$ | $CH_3$ | H | H | H | Cl | Cl | $^1$H-NMR*): 3,85 |
| 7 | 0 | $C_2H_5$ | $C_2H_5$ | H | H | Cl | H | H | $^1$H-NMR*): 4,00 |
| 8 | 0 | $CH_3$ | $CH_3$ | H | H | Cl | H | H | $^1$H-NMR*): 4,05 |

Tabelle 2 - Fortsetzung

| Bsp.-Nr. | n | $R^1$ | $R^2$ | $R^3$ | $R^4$ | $R^5$ | $R^6$ | $R^7$ | Physikalische Daten |
|---|---|---|---|---|---|---|---|---|---|
| 9 | 2 | $C_2H_5$ | $C_2H_5$ | H | H | H | Cl | Cl | $^1$H-NMR*): 4,25 |
| 10 | 2 | $CH_3$ | $CH_3$ | H | H | H | Cl | Cl | Fp.: 86° C |
| 11 | 0 | $C_2H_5$ | $C_2H_5$ | H | H | H | $CH_3$ | Cl | Fp.: 58° C |
| 12 | 2 | $C_2H_5$ | $C_2H_5$ | H | H | Cl | H | H | |
| 13 | 2 | $CH_3$ | $CH_3$ | H | H | Cl | H | H | |
| 14 | 0 | $CH_3$ | $CH_3$ | H | H | H | $CH_3$ | Cl | Fp.: 79° C |
| 15 | 2 | $C_2H_5$ | $C_2H_5$ | H | H | H | $CH_3$ | Cl | |
| 16 | 2 | $CH_3$ | $CH_3$ | H | H | H | $CH_3$ | Cl | Fp.: 76° C |
| 17 | 0 | $CH(CH_3)_2$ | $CH(CH_3)_2$ | H | H | H | Cl | Cl | |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Ausgangsstoffe der Formel (II)

Beispiel (II-1)

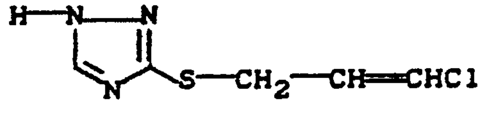

Zu 23,6 g einer technischen Natriummethylat-Lösung (entsprechend 0,11 Mol $NaOCH_3$) in 50 ml Methanol werden unter Eiskühlung 10,1 g (0,10 Mol) 3-Mercapto-1,2,4-triazol gegeben und das Gemisch wird 30 Minuten gerührt. Dann werden - weiter unter Eisbadkühlung - 11,1 g (0,10 Mol) 1,3-Dichlor-propen tropfenweise dazugegeben und das Reaktionsgemisch wird 20 Stunden bei 20 °C gerührt. Nach Verdünnen mit Dichlormethan auf etwa das doppelte Volumen werden ungelöste Anteile durch Absaugen abgetrennt und vom Filtrat wird das Lösungsmittel im Wasserstrahlvakuum sorgfältig abdestilliert.

Man erhält 17,5 g (100% der Theorie) 3-(3-Chlor-2-propen-1-yl-thio)-1H-1,2,4-triazol als öligen Rückstand.

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Verbindungen der Formel (I), insbesondere die gemäß dem Herstellungsbeispiel 2 erhaltene Verbindung, bei guter Verträglichkeit gegenüber Kulturpflanzen, wie z.B. Mais und Sojabohnen, sehr starke Wirkung gegen Unkräuter.

## Ansprüche

1. Substituierte Carbamoyltriazole der allgemeinen Formel (I)

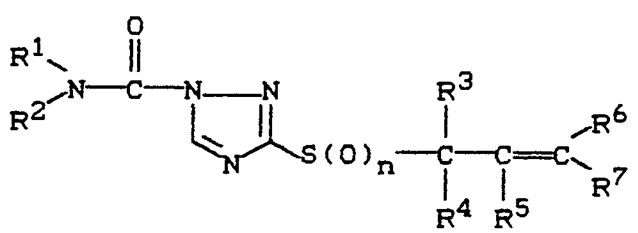

(I)

in welcher

n für die Zahlen 0, 1 oder 2 steht,

$R^1$ und $R^2$ gleich oder verschieden sind und unabhängig voneinander jeweils für geradkettiges oder verzweigtes Alkyl, Alkenyl oder Alkinyl mit jeweils bis zu 6 Kohlenstoffatomen oder zusammen für jeweils geradkettiges oder verzweigtes Alkandiyl oder Oxaalkandiyl mit jeweils bis zu 6 Kohlenstoffatomen stehen,

$R^3$ und $R^4$ gleich oder verschieden sind und unabhängig voneinander jeweils für Wasserstoff oder für geradkettiges oder verzweigtes Alkyl mit bis zu 6 Kohlenstoffatomen stehen, und

$R^5$, $R^6$ und $R^7$ gleich oder verschieden sind und jeweils für Wasserstoff, Halogen, Cyano oder für jeweils geradkettiges oder verzweigtes Alkyl oder Halogenalkyl mit jeweils bis zu 6 Kohlenstoffatomen und gegebenenfalls bis zu 13 Halogenatomen stehen, mit der Maßgabe, daß wenigstens einer der Reste $R^5$, $R^6$ und $R^7$ für Halogen steht.

2. Substituierte Carbamoyltriazole der Formel (I) gemäß Anspruch 1, in welcher

| | |
|---|---|
| n | für die Zahlen 0, 1 oder 2 steht, |
| $R^1$ und $R^2$ | gleich oder verschieden sind und unabhängig voneinander jeweils für geradkettiges oder verzweigtes Alkyl, Alkenyl oder Alkinyl mit jeweils bis zu 4 Kohlenstoffatomen oder zusammen für Tetramethylen (Butan-1,4-diyl), Pentamethylen (Pentan-1,5-diyl) oder 3-Oxapentan-1,5-diyl ($-CH_2CH_2-0-CH_2CH_2-$) stehen, |
| $R^3$ und $R^4$ | gleich oder verschieden sind und unabvoneinander jeweils für Wasserstoff oder für geradkettiges oder verzweigtes Alkyl mit bis zu 3 Kohlenstoffatomen stehen, und |
| $R^5$, $R^6$ und $R^7$ | gleich oder verschieden sind und jeweils für Wasserstoff, Fluor, Chlor, Brom, Jod, Cyano oder für jeweils geradkettiges oder verzweigtes Alkyl oder für durch Fluor und/oder Chlor substituiertes Alkyl mit jeweils bis zu 4 Kohlenstoffatomen und gegebenenfalls bis zu 9 Fluor- und/oder Chloratomen stehen, |

mit der Maßgabe, daß wenigstens einer der Reste $R^5$, $R^6$ und $R^7$ für Fluor, Chlor, Brom oder Jod steht.

3. Substituierte Carbamoyltriazole der Formel (I) gemäß Anspruch 1, in welcher

| | |
|---|---|
| n | für die Zahlen 0, 1 oder 2 steht, |
| $R^1$ und $R^2$ | gleich oder verschieden sind und unabhängig voneinander jeweils für Methyl, Ethyl, Propyl, Isopropyl, Allyl oder Propargyl stehen, |
| $R^3$ | für Wasserstoff oder Methyl steht, |
| $R^4$ | für Wasserstoff, Methyl, Ethyl, Propyl oder Isopropyl steht, |
| $R^5$ | für Wasserstoff, Chlor, Methyl oder Ethyl steht, und |
| $R^6$ und $R^7$ | gleich oder verschieden sind und jeweils für Wasserstoff, Chlor, Methyl, Ethyl oder Propyl stehen, |

mit der Maßgabe, daß wenigstens einer der Reste $R^5$, $R^6$, $R^7$ für Chlor steht.

4. Verfahren zur Herstellung von substituierten Carbamoyltriazolen der Formel (I)

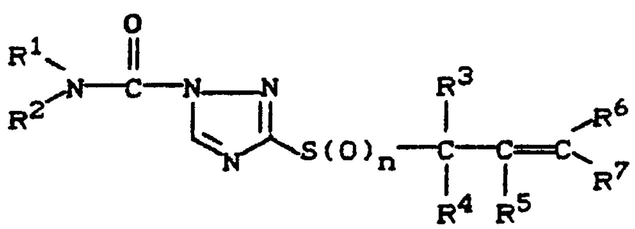

(I)

in welcher

| | |
|---|---|
| n | für die Zahlen 0, 1 oder 2 steht, |
| $R^1$ und $R^2$ | gleich oder verschieden sind und unabhängig voneinander jeweils für geradkettiges oder verzweigtes Alkyl, Alkenyl oder Alkinyl mit jeweils bis zu 6 Kohlenstoffatomen oder zusammen für jeweils geradkettiges oder verzweigtes Alkandiyl oder Oxaalkandiyl mit jeweils bis zu 6 Kohlenstoffatomen stehen, |
| $R^3$ und $R^4$ | gleich oder verschieden sind und unabhängig voneinander jeweils für Wasserstoff oder für geradkettiges oder verzweigtes Alkyl mit bis zu 6 Kohlenstoffatomen stehen, und |
| $R^5$, $R^6$ und $R^7$ | gleich oder verschieden sind und jeweils für Wasserstoff, Halogen, Cyano oder für jeweils geradkettiges oder verzweigtes Alkyl oder Halogenalkyl mit jeweils bis zu 6 Kohlenstoffatomen und gegebenenfalls bis zu 13 Halogenatomen stehen, |

mit der Maßgabe, daß wenigstens einer der Reste $R^5$, $R^6$ und $R^7$ für Halogen steht,
dadurch gekennzeichnet, daß man
(a) substituierte Triazole der allgemeinen Formel (II)

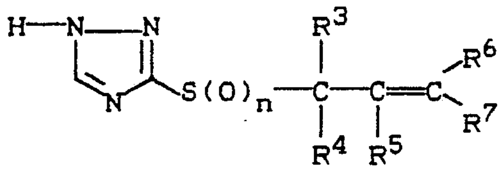

(II)

in welcher
n, $R^3$, $R^4$, $R^5$, $R^6$ und $R^7$ die oben angegebenen Bedeutungen haben,
oder Tautomere zu den Verbindungen der Formel (II) mit Carbamidsäurechloriden der allgemeinen Formel (III)

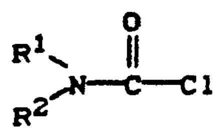

(III)

in welcher
$R^1$ und $R^2$ die oben angegebenen Bedeutungen haben,
gegebenenfalls in Gegenwart eines Säureakzeptors und gegebenenfalls in Gegenwart eines Verdünnungsmittels umsetzt, oder daß man
(b) substituierte Carbamoyltriazole der allgemeinen Formel (Ia)

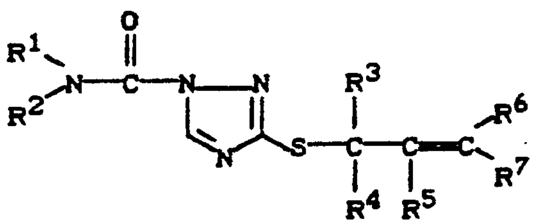

(Ia)

in welcher
$R^1$, $R^2$, $R^3$, $R^4$, $R^5$, $R^6$ und $R^7$ die oben angegebenen Bedeutungen haben,
mit Oxidationsmitteln, gegebenenfalls in Gegenwart von Verdünnungsmitteln umsetzt.

**5.** Herbizide Mittel, gekennzeichnet durch einen Gehalt an mindestens einem substituierten Carbamoyltriazol der Formel (I) gemäß Anspruch 1 oder 4.

**6.** Verfahren zur Bekämpfung von unerwünschten Pflanzen, dadurch gekennzeichnet, daß man substituierte Carbamoyltriazole der Formel (I) gemäß Anspruch 1 oder 4 auf die Pflanzen und/oder ihren Lebensraum einwirken läßt.

**7.** Verwendung von substituierten Carbamoyltriazolen der Formel (I) gemäß Anspruch 1 oder 4 zur Bekämpfung von unerwünschten Pflanzen.

**8.** Verfahren zur Herstellung von herbiziden Mitteln, dadurch gekennzeichnet, daß man substituierte Carbamoyltriazole der Formel (I) gemäß Anspruch 1 oder 4 mit Streckmitteln und/oder oberflächenaktiven Substanzen vermischt.

**9.** Substituierte Triazole der Formel (II)

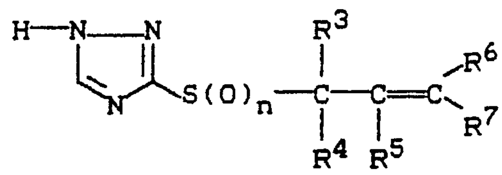 (II)

in welcher

n für die Zahlen 0, 1 oder 2 steht,

$R^3$ und $R^4$ gleich oder verschieden sind und unabhängig voneinander jeweils für Wasserstoff oder für geradkettiges oder verzweigtes Alkyl mit bis zu 6 Kohlenstoffatomen stehen, und

$R^5$, $R^6$ und $R^7$ gleich oder verschieden sind und jeweils für Wasserstoff, Halogen, Cyano oder für jeweils geradkettiges oder verzweigtes Alkyl oder Halogenalkyl mit jeweils bis zu 6 Kohlenstoffatomen und gegebenenfalls bis zu 13 Halogenatomen stehen,

mit der Maßgabe, daß wenigstens einer der Reste $R^5$, $R^6$ und $R^7$ für Halogen steht.

Europäisches Patentamt

# EUROPÄISCHER RECHERCHENBERICHT

Nummer der Anmeldung

EP 90123603.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.5) |
|---|---|---|---|
| D,A | DE - A1 - 2 132 618 (BOOTS) * Ansprüche 1,12; Formel III; Tabelle I * -- | 1,5,9 | C 07 D 249/12<br>C 07 D 413/06<br>C 07 D 401/06<br>A 01 N 43/653<br>A 01 N 43/84 |
| A | US - A - 3 983 113 (BEEBY) * Beispiele 2-14 * -- | 1,7,9 | |
| A | US - A - 4 255 435 (WATKINS) * Formel IV * -- | 1,9 | |
| A | FR - A - 2 386 969 (BOOTS) * Formel II,III; Beispiel 5; Seite 7, Zeile 19; Anspruch 16 * -- | 1,5,7,9 | |
| A | DE - A1 - 2 636 605 (SYNTEX) * Beispiele 3-8 * -- | 1,9 | RECHERCHIERTE SACHGEBIETE (Int Cl.5) |
| A | DE - A1 - 2 708 330 (GLAXO) * Beispiele 9,94,104 * ---- | 1,7,9 | C 07 D 249/00<br>C 07 D 413/00<br>C 07 D 401/00 |

Der vorliegende Recherchenbericht wurde für alle Patentansprüche erstellt.

| Recherchenort | Abschlußdatum der Recherche | Prüfer |
|---|---|---|
| WIEN | 01-02-1991 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument